# EUROPEAN PATENT SPECIFICATION

(11) **EP 0 848 757 B1**
(45) Date of publication and mention of the grant of the patent: **06.03.2002**
(21) Application number: 96931040.8
(22) Date of filing: 06.09.1996
(51) Int. Cl.: C12N 15/867, A61K 48/00, C12N 15/85, C12N 5/10

(54) **THE USE OF THE WAP OF MMTV REGULATORY SEQUENCES FOR TARGETED EXPRESSION OF LINKED HETEROLOGOUS GENES IN HUMAN MAMMARY CELLS, INCLUDING HUMAN MAMMARY CARCINOMA CELLS**
EXPRESSION VON HETEROLOGEN GENEN IN MENSCHLICHEN BRUSTZELLEN, EINSCHLIESSLICH MENSCHLICHE BRUSTKARZIONOMZELLEN UNTER DER KONTROLLE VON REGULATORISCHEN SEQUENZEN VON WAP ODER MMTV
UTILISATION DE SEQUENCES REGULATRICES DE LA PROTEINE ACIDE DU LACTOSERUM OU DU VIRUS DE LA TUMEUR MAMMAIRE DE LA SOURIS, AUX FINS D'EXPRESSION CIBLEE DE GENES HETEROLOGUES LIES DANS DES CELLULES MAMMAIRES HUMAINES ET NOTAMMENT DANS DES CELLULES DU CANCER MAMMAIRE HUMAIN

(30) Priority: 06.09.1995 DK 97695
(43) Date of publication of application: 24.06.1998
(73) Proprietor: Austrian Nordic Biotherapeutics AG, 1210 Wien (AT); GSF - Forschungszentrum für Umwelt und Gesundheit, GmbH, 85758 Oberschleissheim (DE)
(72) Inventor: GÜNZBURG, Walter, H., D-85229 Ainhofen (DE); SALLER, Robert, Michael, D-80636 München (DE); SALMONS, Brian, D-85229 Ainhofen (DE)
(74) Representative: Pielken, Petra, Dr.
(86) International application number: EP9603922
(87) International publication number: WO9709440

(56) References cited:
- WO-A-94/05796
- WO-A-95/15334
- BIOLOGICALS, vol. 23, no. 1, March 1995, pages 5-12, XP002022643 W.H.GÜNZBURG ET AL.: "Retroviral vectors directed to predefined cell types for gene therapy"

## Description

The present invention relates to the use of the rodent WAP (Whey Acidic Protein) and the MMTV (Mouse Mammary Tumor Virus) regulatory sequences for targeted expression of linked heterologous genes, in particular therapeutic genes in human mammary cells, including human mammary carcinoma cells.

### Background of the Invention

Mammary carcinoma is the most frequent tumour in women (Miller and Bulbrook, 1986). Up to now conventional therapy involves surgical removal of the primary tumour followed by a chemo- or radiation therapy. Depending on the tumour stage, the rate of relapse is quite high and has a fatal outcome in most cases.

A major problem is the elimination of all metastases and micrometastases. Both this, as well as the serious side effects for the patient caused by conventional treatment, favour the development of a gene therapy approach (for a review on gene therapy see Anderson, 1992). A gene therapeutic approach however poses the problem of targeting the expression of the therapeutic gene to tumor cells. A control element is therefore required to ensure that the therapeutic gene is only expressed in tumor cells.

Vector constructs carrying various types of mammary gland specific regulatory elements have been tested in mice where expression of a marker gene driven by the regulatory elements in the hormonally stimulated mammary gland could be achieved (WO-A1-9607748). One regulatory element demonstrated to give rise to expression in the pregnant and lactating mouse mammary gland is a small region of the rodent WAP promoter (Kolb et al., 1994). This gene is only expressed in the pregnant and lactating mammary glands of rodents and has no human homologue (Hennighausen, 1992). It is therefore not predictable that this regulatory element will function at all to direct expression in human mammary cells and/or allow expression in human mammary carcinoma cells.

It was thus quite unexpected when the inventors of the present invention found that the WAP regulatory sequence is able to direct expression of a linked heterologous gene in primary human cells, including mammary carcinoma cells.

### Summary of the Invention

The invention then, inter alia, comprises the following, alone or in combination:
A DNA construct comprising at least one therapeutic gene under transcriptional control of the WAP or MMTV regulatory sequences for the treatment of disorders or diseases of human mammary cells, including human mammary carcinoma;
a DNA construct as above wherein the regulatory sequence comprises the proximal 445 bp of the WAP promoter including the transcription initiation site;
a DNA construct as above wherein the regulatory sequence contain the 320 bp XhoI/Xbal fragment of the WAP promoter region;
a DNA construct as above wherein the regulatory sequence is the U3 region of MMTV;
a DNA construct as above wherein the regulatory sequence contain the 0.6 Kb Pstl MMTV promoter fragment;
a DNA construct as above which is a recombinant vector selected from viral and plasmid vectors;
a recombinant vector as above wherein said viral vector is selected
   from RNA and DNA viral vectors and said plasmid vector is selected from eucaryotic expression vectors;
a recombinant vector as above wherein said viral vector is a retroviral vector, a recombinant adenovirus vector, a recombinant adeno-associated virus vector or a recombinant herpes virus vector;
a recombinant vector as above wherein the retroviral vector comprises a 5'LTR region of the structure U3-R-U5; at least one coding sequence coding for a therapeutic gene; and a 3' LTR region comprising a completely or partially deleted U3 region wherein said deleted region has been replaced by a polylinker containing the WAP or MMTV regulatory sequences followed by the R and U5 region, said therapeutic gene being under transcriptional control of the WAP or MMTV regulatory sequences;
a construct as above wherein the therapeutic gene is selected from anti-tumor genes and cytokine genes for the treatment of human mammary carcinoma;
a construct as above, wherein said therapeutic gene is selected from the group consisting of genes which code for proteins such as Herpes Simplex Virus thymidine kinase, cytosine deaminase, guanine phosphoribosyl transferase (gpt), cytochrome P 450, cell cycle regulatory genes which code for proteins such as SDI, tumor supressor genes which code for proteins such as p53, antiproliferation genes which codes for proteins such as melittin, cecropin or cytokines such as IL-2;
a recombinant retroviral particle produced by culturing a packaging cell line harbouring a retroviral vector construct as above and one or more constructs coding for the proteins required for the genome of said retroviral vector to be packaged, for the treatment of disorders or diseases of human mammary cells, including human mammary carcinoma;
a retroviral provirus carrying a construct comprising at least one therapeutic gene under transcriptional control of the WAP or MMTV regulatory sequences integrated in the human genome;
a retroviral provirus as above comprising a 5'LTR region comprising a completely or partially deleted U3 region wherein said deleted region has been replaced by a polylinker containing the WAP or MMTV regulatory sequences followed by the R and U5 region; at least one coding sequence coding for a therapeutic gene; and a 3' LTR region comprising a completely or partially deleted U3 region wherein said deleted region has been replaced by a polylinker containing the WAP or MMTV regulatory sequences followed by the R and U5 region, said therapeutic gene being under transcriptional control of the WAP or MMTV regulatory sequences;
a cell line containing a construct as above for the treatment of disorders or diseases of human mammary cells, including human mammary carcinoma;
a cell line as above which is a packaging cell line harbouring a retroviral vector construct as above and one or more constructs coding for the proteins required for the genome of said retroviral vector to be packaged;
a human cell containing a retroviral provirus as above.
encapsulated cells comprising a core containing cells as above and a porous capsule wall surrounding said core, said porous capsule wall being permeable to the therapeutic polypeptide or the viral particles produced by said cells for the treatment of disorders or diseases of human mammary cells, including human mammary carcinoma;
encapsulated cells as above wherein said porous capsule wall consists of a polyelectrolyte complex formed from counter charged polyelectrolytes;
the use of a construct as above for the preparation of a medicament for the treatment of disorders or diseases of human mammary cells, including human mammary carcinoma;
the use of a recombinant viral particle as above for the manufacture of a medicament for the treatment of disorders or diseases of human mammary cells, including human mammary carcinoma;
the use of cells as above for the manufacture of a medicament for the treatment of a disorder or disease of human mammary cells, including human mammary carcinoma;
a pharmaceutical composition for the treatment of disorders or diseases of human mammary cells, including human mammary carcinoma comprising a
   DNA construct as above and a pharmaceutically acceptable carrier or diluent;
a pharmaceutical composition for the treatment of disorders or diseases of human mammary cells, including human mammary carcinoma comprising a
   a recombinant retroviral particle as above and a pharmaceutically acceptable carrier or diluent;
a pharmaceutical composition for the treatment of disorders or diseases of human mammary cells, including human mammary carcinoma comprising a
   a cell line as above and a pharmaceutically acceptable carrier or diluent;
the use of the WAP or MMTV regulatory sequences for the expression of linked therapeutic genes in human mammary cells, including human mammary carcinoma cells;
the use as above wherein the regulatory sequence comprises the proximal 445 bp of the WAP promoter including the transcription initiation site;
the use as above wherein the regulatory sequence contain the 320 bp Xhol/Xbal fragment of the WAP promoter region;
the use as above wherein the regulatory sequence is the U3 region of MMTV;
the use as above wherein the regulatory sequence contain the 0.6 Kb Pstl MMTV promoter fragment;
the use as above wherein the therapeutic gene is selected from anti-tumor genes and cytokine genes;
the use as above, wherein said therapeutic gene is selected from the group consisting of genes which code for proteins such as Herpes Simplex Virus thymidine kinase, cytosine deaminase, guanine phosphoribosyl transferase (gpt), cytochrome P 450, cell cycle regulatory genes which code for proteins such as SDI, tumor supressor genes which code for proteins such as p53, antiproliferation genes which codes for proteins such as melittin, cecropin or cytokines such as IL-2;
the use as above wherein the therapeutic gene under transcriptional control of the WAP or MMTV regulatory sequences form part of a recombinant vector selected from viral and plasmid vectors;
the use as above wherein said viral vector is selected from RNA and DNA viral vectors and said plasmid vector is selected from eucaryotic expression vectors;
the use as above wherein said viral vector is a retroviral vector;
the use as above wherein the retroviral vector comprises a 5'LTR region of the structure U3-R-U5; at least one coding sequence coding for a therapeutic gene; and a 3' LTR region comprising a completely or partially deleted U3 region wherein said deleted region has been replaced by a polylinker containing the WAP or MMTV regulatory sequences followed by the R and U5 region, said therapeutic gene being under transcriptional control of the WAP or MMTV regulatory sequences;
a method for the treatment of human mammary carcinoma comprising administering to a human in need thereof a construct as above;
a method for the treatment of human mammary carcinoma comprising administering to a human in need thereof a viral particle as above;
a method for the treatment of human mammary carcinoma comprising administering to a human in need thereof cells as above; and
a method for the treatment of human mammary carcinoma comprising implanting into a human in need thereof encapsulated cells as above either in or nearby the site of the tumor.

The therapeutic gene is preferably selected from one or more elements of the group consisting of antitumor genes and cytokine genes.

Said therapeutic genes are preferably selected from the group consisting of genes which codes for proteins such as Herpes Simplex Virus thymidine kinase, cytosine deaminase, guanine phosphoribosyl transferase (gpt), cytochrome P 450, cell cycle regulatory genes such as SDI, or tumor supressor genes which codes for proteins such as p53, or antiproliferation genes which codes for proteins such as melittin, cecropin or cytokines such as IL-2.

Herpes Simplex Virus thymidine kinase, cytosine deaminase, guanine phosphoribosyl transferase (gpt) and cytochrome P 450 can be used in cancer treatment in combination with a prodrug which is converted to its toxic form by these enzymes.

The DNA constructs according to the invention can also carry a marker gene.
Said marker genes are preferably selected from the group consisting of marker genes which codes for proteins such as β-galactosidase, neomycin, alcohol dehydrogenase, luciferase, puromycin, hypoxanthine phosphoribosyl transferase (HPRT), hygromycin, secreted alkaline phosphatase and green or blue fluoroscent proteins.

According to the invention it has surprisingly been found that the WAP and MMTV regulatory sequences are able to direct expression of a linked heterologous gene in primary human mammary cells, including human mammary carcinoma cells.

It is well known that Whey Acidic Protein (WAP) gene expression is directed to the pregnant and lactating mammary gland of rodents (Günzburg et al., 1991, Hennighausen, 1992). WAP gene expression is regulated both by hormone dependent and hormone independent mechanisms. A negative regulatory element (NRE) in the WAP promoter interacts with a NRE binding factor, NBF, which is present in all cells unable to express WAP (Kolb et al, 1995). It appears that the region of the WAP promoter which is required for mediating the mammary gland specificity is a 320 bp XhoI/XbaI restriction fragment (-413 to -93) (Kolb et al., 1995). In addition certain experiments indicate that binding sites for NBF is present on a 0.6 Kb Pstl MMTV promoter fragment (described in Salmons et al., 1985) and may play a role in regulating the mammary gland specificity of expression displayed by MMTV (Kolb et al., 1995).

The fact that the WAP and the MMTV are both derived from the rodent system may become an important safety feature because the use of human regulatory sequences in a retroviral vector may facilitate homologous recombinations between the vector carried sequences and the corresponding cellular sequence and may cause genome instability.

The DNA constructs according to the invention carrying a therapeutic gene under transcriptional control of the WAP or MMTV regulatory sequences can be introduced into cells using any known method for the introduction of genes into cells including by means of calcium phospate precipitation (Graham et al., 1973, Wigler et al., 1979) by means of electroporation (Neumann et al., 1982), by microinjection (Graessmann et al., 1983), by means of liposomes (Straubinger et al., 1983), by means of spheroblasts (Schaffner, 1980) or by other methods known to those skilled in the art.

The DNA constructs according to the invention carrying a therapeutic gene under transcriptional control of the WAP or MMTV regulatory sequences are preferably introduced into cells using a recombinant viral vector. Such vectors are well known in the art and includes retroviral vectors, recombinant adenoviruses and recombinant adeno-associted viruses (Günzburg and Salmons, 1995) as well as herpes virus vectors.

In a preferred embodiment of the invention the DNA construct according to the invention is a modified retroviral vector, which is used to deliver the therapeutic gene to cells, preferably in vivo. The great advantage of a retroviral system is that retroviruses can only infect dividing cells (especially rapid dividing cells such as tumor cells) and that the virus particles can be spread in the blood stream similarly to metastasising tumour cells, which will make it possible to eliminate micrometastases long before they can be detected by conventional methods.

Retroviral vector systems consist of two components:
1) the retroviral vector itself is a modified retrovirus (vector plasmid) in which the genes encoding for the viral proteins have been replaced by therapeutic genes and/or marker genes to be transferred to the target cell. Since the replacement of the genes encoding for the viral proteins effectively cripples the virus it must be rescued by the second component in the system which provides the missing viral proteins to the modified retrovirus.

The second component is:
2) a cell line that produces large quantities of the viral proteins, however lacks the ability to produce replication competent virus. This cell line is known as the packaging cell line and consists of a cell line transfected with one or more plasmids carrying the genes enabling the modified retroviral vector to be packaged.

To generate the packaged vector, the vector plasmid is transfected into the packaging cell line. Under these conditions the modified retroviral genome including the inserted therapeutic and marker genes is transcribed from the vector plasmid and packaged into the modified retroviral particles (recombinant viral particles). This recombinant virus is then used to infect target cells in which the vector genome and any carried marker or therapeutic genes become integrated into the target cell's DNA. A cell infected with such a recombinant viral particle cannot produce new vector virus since no viral proteins are present in these cells. However the DNA of the vector carrying the therapeutic and marker genes is integrated in the cell's DNA as a provirus and can now be expressed in the infected cell.

WO-A1-9607748 describes the principle and construction of a new type of retroviral vector, the ProCon-vector:

The retroviral genome consists of an RNA molecule with the structure R-U5-gag-pol-env-U3-R. During the process of reverse transcription, the U5 region is duplicated and placed at the right hand end of the generated DNA molecule, whilst the U3 region is duplicated and placed at the left hand end of the generated DNA molecule. The resulting terminal structure U3-R-U5 is called LTR (Long Terminal Repeat) and is thus identical and repeated at both ends of the DNA structure or provirus (Varmus, 1988). The U3 region at the left hand end of the provirus harbours the promoter that is used to drive expression after infection has occured. This promoter drives the synthesis of an RNA transcript initiating at the boundary between the left hand U3 and R regions and terminating at the boundary between the right hand R and U5 region. This RNA is packaged into retroviral particles and transported into the target cell to be infected. In the target cell the RNA genome is reverse transcribed as described above.

In the ProCon-vector plasmid the right-hand (3') U3 region is altered, but the normal left-hand (5') U3 structure is maintained; the vector can be normally transcribed into RNA utilizing the normal retroviral promoter located within the left-hand (5') U3 region upon its introduction into packaging cells. However the generated RNA will only contain the altered right-hand (3') U3 structure. In the infected target cell, after reverse transcription, this altered U3 structure will be present in both Long Terminal Repeat at either end of the retroviral structure.

If the altered region carries a polylinker instead of the U3 region then any promoter, including those directing tissue specific expression such as the WAP regulatory sequences can easily be inserted. This promoter can then be utilized exclusively in the target cell for expression of linked genes carried by the retroviral vector. Additionally DNA segments homologous to one or more celluar sequences can be inserted into the polylinker for the purposes of gene targeting, by homologous recombination.

The retroviral vectors of the invention need not be of the ProCon type, but can be any conventional retroviral vector carrying therapeutic genes under transcriptional control of the WAP or MMTV regulatory sequences.

Such vectors include Self-Inactivating-Vectors (SIN) in which retroviral promoters are functionally inactivated in the target cell (WO-A1-94/29437). Further modifications of these vectors include the insertion of promoter gene cassettes within the LTR region to create double copy vectors (WO-A1-89/11539). In both of these vectors the heterologous promoters inserted either in the body of the vector, or in the LTR region are directly linked to the therapeutic gene.

The vector of the invention is preferably a retroviral vector wherein the WAP or MMTV promoters are used as internal promoters, i.e. LTR-neo-*WAP-tk*-LTR, but most preferably the retroviral vector of the invention is of the ProCon type.

The retroviral vector is based preferably either on a BAG vector (Price *et al*., 1987) or an LXSN vector (Miller and Rosman, 1989), or derivatives thereof.

Retroviral particles can be made by introducing a viral vector construct into a packaging cell which contain the elements lacking in the vector construct (e.g. gag, pol, and env) which are necessary for the production of infectious retroviruses.

The packaging cell line can be selected from an element of the group consisting of psi-2 (Mann et al., 1983), psi-Crip (Danos and Mulligan, 1988) psi-AM (Cone and Mulligan 1984), GP+E-86 ( Markowitz et al., 1988a), PA317 (Miller and Buttimore, 1986), GP+envAM-12 (Markowitz et al., 1988b), Bosc 23, Bing ( Pear et al., 1993) or FLYA13, FLYRD18 (Casset et al., 1995) or of any of these transfected with recombinant constructs allowing expression of surface proteins from other enveloped viruses. Such pseudotyped retroviral particles are described in PCT/EP96/01348.

In a particular preferred embodiment, the packaging cell line is made from human cells, e.g. HT1080 cells (WO-A1-9621014), 293 (Graham et al., 1977) or mink cell lines thereby allowing production of recombinant retroviruses that are capable of surviving inactivation by human serum.

According to the invention the term "polylinker" is used for a short stretch of artificially synthesized DNA which carries unique restriction sites allowing the easy insertion of any promoter or DNA segment.

The term "heterologous" is used for any combination of DNA sequences that is not normally found intimately associated in nature.

### Preservation and Administration of Viral Particles

Recombinant retrovirus which has been purified or concentrated may be preserved by first adding a sufficient amount of a formulation buffer to the media containing the recombinant retrovirus, in order to form an aqueous suspension. The formulation buffer is an aqueous solution that contains a saccharide, a high molecular weight structural additive, and a buffering component in water. The aqueous solution may also contain one or more amino acids.

The recombinant retrovirus can also be preserved in a purified form. More specifically, prior to the addition of the formulation buffer, the crude recombinant retrovirus described above may be clarified by passing it through a filter, and then concentrated, such as by a cross flow concentrating system (Filtron Technology Corp., Nortborough, MA). Within one embodiment, DNase is added to the concentrate to digest exogenous DNA. The digest is then diafiltrated to remove excess media components and establish the recombinant retrovirus in a more desirable buffered solution. The diafiltrate is then passed over a Sephadex S-500 gel column and a purified recombinant retrovirus is eluted. A sufficient amount of formulation buffer is added to this eluate to reach a desired final concentration of the constituents and to minimally dilute the recombinant retrovirus, and the aqueous suspension is then stored, preferably at -70°C or immediately dried. As noted above, the formulation buffer is an aqueous solution that contains a saccharide, a high molecular weight structural additive, and a buffering component in water. The aqueous solution may also contain one or more amino acids.

The crude recombinant retrovirus can also be purified by ion exchange column chromatography. In general, the crude recombinant retrovirus is clarified by passing it through a filter, and the filtrate loaded onto a column containing a highly sulfonated cellulose matrix. The recombinant retrovirus is eluted from the column in purified form by using a high salt buffer. The high salt buffer is then exchanged for a more desirable buffer by passing the eluate over a molecular exclusion column. A sufficient amount of formulation buffer is then added, as discussed above, to the purified recombinant retrovirus and the aqueous suspension is either dried immediately or stored, preferably at -70°C.

The aqueous suspension in crude or purified form can be dried by lyophilisation or evaporation at ambient temperature. Specifically, lyophilisation involves the steps of cooling the aqueous suspension below the glass transition temperature or below the eutectic point temperature of the aqueous suspension, and removing water from the cooled suspension by sublimation to form a lyophilised retrovirus. Once lyophilised, the recombinant retrovirus is stable and may be stored at -20°C to 25°C, as discussed in more detail below.

Within the evaporative method, water is removed from the aqueous suspension at ambient temperature by evaporation. Water can also be removed through spray drying.

The aqueous solutions used for formulation, as previously described, are composed of a saccharide, high molecular weight structural additive, a buffering component, and water. The solution may also include one or more amino acids. The combination of these components act to preserve the activity of the recombinant retrovirus upon freezing and lyophilization, or drying through evaporation.

The high molecular weight structural additive aids in preventing viral aggregation during freezing and provides structural support in the lyophilised or dried state. Within the context of the present invention, structural additives are considered to be of "high molecular weight" if they are greater than 5000 m.w. A preferred high molecular weight structural additive is human serum albumin.

The amino acids, if present, function to further preserve viral infectivity upon cooling and thawing of the aqueous suspension. In addition, amino acids function to further preserve viral infectivity during sublimation of the cooled aqueous suspension and while in the lyophilised state.

The buffering component acts to buffer the solution by maintaining a relatively constant pH. A variety of buffers may be used, depending on the pH range desired, preferably between 7.0 and 7.8.

Aqueous solutions for the formulation of recombinant retroviruses are described in detail in WO-A2-96121014.

In addition, it is preferable that the aqueous solution contain a neutral salt which is used to adjust the final formulated recombinant retrovirus to an appropriate iso-osmotic salt concentration.

Lyophilized or dehydrated retroviruses may be reconstituted using a variety of substances, but are preferably reconstituted using water. In certain instances, dilute salt solutions which bring the final formulation to isotonicity may also be used. In addition, it may be advantageous to use aqueous solutions containing components known to enhance the activity of the reconstituted retrovirus. Such components include cytokines, such as IL-2, polycations, such as protamine sulfate, or other components which enhance the transduction efficiency of the reconstituted retrovirus. Lyophilized or dehydrated recombinant retrovirus may be reconstituted with any convenient volume of water or the reconstituting agents that allow substantial, and preferably total solubilization of the lyophilized or dehydrated sample.

Recombinant retroviral particles may be administered to a wide variety of locations including, for example, into sites such as an organ or to a site of a tumor. Within other embodiments, the recombinant retrovirus may be administered orally, intravenously, buccal/sublingual, intraperitoneally, or subcutaneously. The daily dosage depends upon the exact mode of administration, form in which administered, the indication toward which the administration is directed, the subject involved and the body weight of the subject involved, and further the preference and experience of the physician in charge.

The routes of administration described herein may be accomplished simply by direct administration using a needle, catheter or related device. In particular, within certain embodiments of the invention, one or more dosages may be administered directly.

In another embodiment of the invention a cell line according to the invention producing either a therapeutic polypeptide or viral particles carrying a therapeutic gene under the transcriptional control of the WAP or MMTV regulatory sequences is encapsulated in a porous membrane which is permeable to the therapeutic polypeptide or viral particles produced (see Stange et al., 1993, Dautzenberg et al.,1993, Merten et al., 1991 and UK patent application 2 135 954).

The encapsulated cells according to the invention can be prepared for example by suspending the cells in an aqueous solution of a polyelectrolyte (e.g. selected from sulphate group-containing polysaccharides or polysaccharide derivatives or of sulphonate group containing synthetic polymers), whereafter the suspension in the form of preformed particles is introduced into a precipitation bath containing an aqueous solution of a counter-charged polyelectrolyte (such as for example a polymer with quaternary ammonium groups).

Sulphate group-containing polysaccharides or polysaccharide derivatives includes cellulose sulphate, cellulose acetate sulphate, carboxymethylcellulose sulphate, dextran sulphate or starch sulphate in the form of a salt, especially a sodium salt.
The sulphonate group-containing synthetic polymer can be a polystyrene sulphonate salt, preferably a sodium salt.

Polymers with quaternary ammonium groups includes polydimethyldiallylammonium or polyvinylbenzyl-trimethylammonium, in the form of a salt thereof, preferably a cloride salt.

Such capsules are preferably prepared by suspending the cells of the invention in a solution containing 0.5-50%, preferably 2-5%, sodium cellulose sulphate and 5% fetal calf serum in PBS. This suspension is then dropped by a dispensing system (e.g. air-jet system or piezoelectric system) while stirring into a precipitation bath containing 0.5%-50%, preferably 2-10%, or most prefered 3% polydimethyl-diallylammonium chloride in PBS. Capsule formation occurs within milliseconds and the capsules containing cells are kept in the precipitation bath for 30 seconds to 5 minuntes and then washed. The rapidity of this method ensures that the cells are not unduly stressed during the whole procedure (Stange et al., 1993).

The encapsulated cells can be cultivated in a normal cell culture medium (the nature of which depends on the encapsulated cells) at standard conditions of humidity, temperature and CO₂ concentration. During this culture period production of therapeutic polypeptides and viral particles from the capsules into the cell culture medium can be demonstrated. Production of viral particles can be demonstrated using for RT-PCR technology or by transfer of cell free (0.45 µm filtered) supernatant to target cells followed by the demonstration of viral infection by assay for the activity of marker proteins encoded by genes carried by the viral vector construct contained within the viral particle. If the marker gene carried by the viral vector is a gene confering resistance to a specific compound upon the target cell or the product of which is easily assayed on a cell to cell basis e.g green or blue fluoroscent protein, the titre of virus produced by the system can be acertained.

After a suitable period in culture (normally not less than 1 hour and not exceeding 30 days), the cell containing capsules can be surgically implanted either directly, or by injection using a syringe into various areas of the body including the breast.

### Drawings

- Figure 1:: Primers A, B, and C used for deletion of U3 of MLV and insertion of a Sacll-Mlu polylinker in its place.
- Figure 2:: The preparation of plasmid pSmaU3del.
- Figure 3:: The preparation of plasmids pBAGNU3del and pCON6
- Figure 4:: The preparation of plasmid pMMTV-BAG.
- Figure 5:: Primers D and E used for amplification of the U3-region of MMTV.
- Figure 6:: Plasmid pMMTV-BAG.
- Figure 7:: Primers F and G used for amplification of a sequence containing the proximal 445 bp of the WAP promoter and the first 143 bp's of human growth hormone.
- Figure 8:: The preparation of plasmid pWAP-BAG
- Figure 9:: Plasmid pWAP-BAG
- Figure 10:: β-Gal expression of vector constructs after infection of primary human mammary gland cells.

The following examples will illustrate the invention further. The example is however in no way intended to limit the scope of the present invention as obvious modifications will be apparent, and still other modifications and substitutions will be apparent to anyone skilled in the art.

The recombinant DNA methods employed in practicing the present invention are standard procedures, well known to those skilled in the art, and described in detail, for example, in "Molecular Cloning" (Sambrook et al. 1989) and in "A Practical Guide to Molecular Cloning" (Perbal, 1984).

### Example 1

### Deletion of the U3 region and insertion of a polylinker

In the murine leukemia virus (MLV) retroviral vector known as BAG (Price et al., 1987) the β-galactosidase gene is driven by the promiscuous (i.e. non-tissue specific) MLV promoter in the U3 region of the LTR. According to the present invention a derivative of the BAG vector has been constructed in which the MLV promoter (U3) located within the 3'LTR except the inverted repeat has been deleted by PCR and replaced by a polylinker. The BAG vector lacking the U3 is expressed from the MLV promoter (U3) within the 5'LTR when introduced into a packaging cell line. As a result of the rearrangements occurring in the retroviral genome during its life cycle, following infection of its target cell, the polylinker will be duplicated at both ends of the retroviral genome as described in WO-A1-9607748. Thereby a retroviral vector can be constructed in which the expression of the β-galactosidase gene of BAG will be controlled by any heterologous promoter inserted into the polylinker.

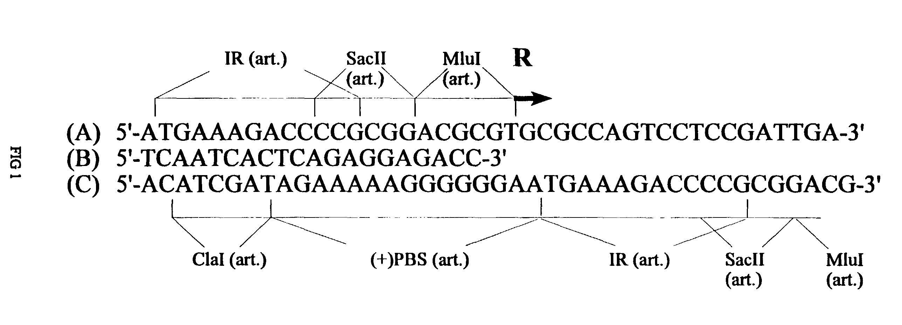

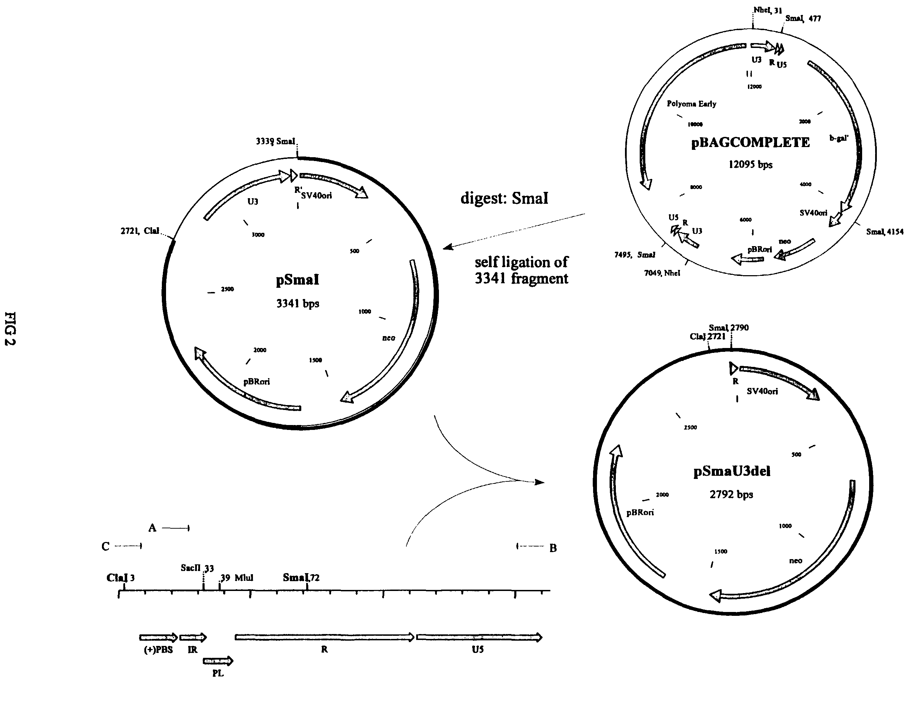

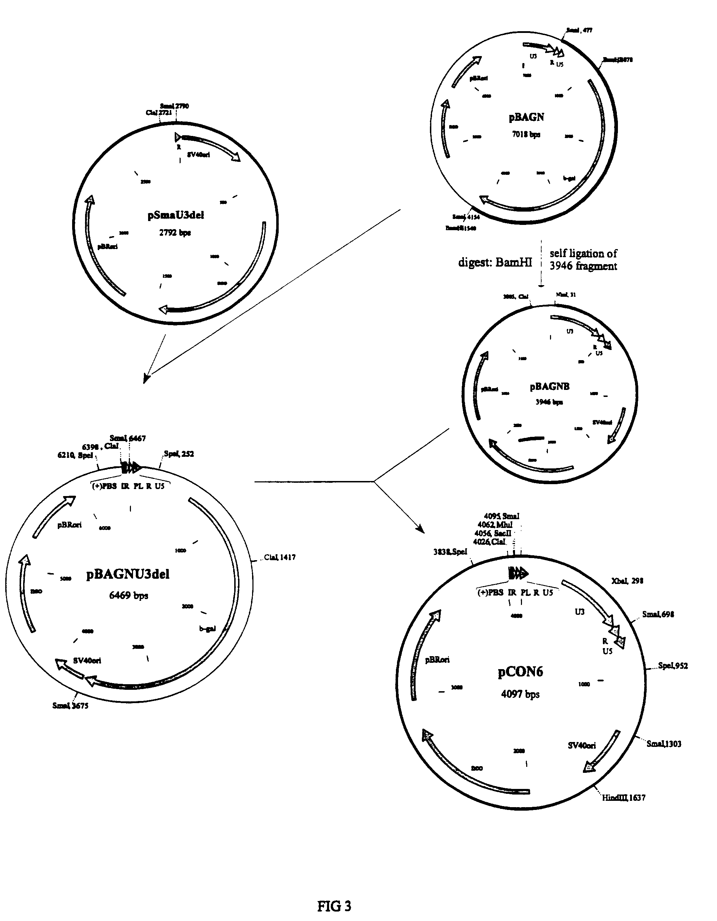

As a template for PCR we used pBAGN a plasmid carrying a derivative of the BAG construct carrying only one LTR, created by an Nhel digest of the original pBAG followed by a self-ligation of the 7018bp fragment.
The 3' end of primer A is complementary to the R-region of the LTR (Fig. 1). The 5'-extension contains an artificial (art.) polylinker and an artificial inverted repeat (IR(art.)). Primer B is complementary to the U5 region of the LTR (Fig. 1). After 35 cycles of annealing at 47°C and extension at 60°C, a 140bp product was obtained, which was used as a template for the second PCR. In this reaction a Clal site and an artificial (+)PBS was added 5'of the IR-region using primer C (Fig. 1) in combination with primer B. Annealing was carried out at 53°C and extension at 72°C. After 35 cycles a 163bp product was obtained, which was digested with Clal and Smal and ligated to a 2722bp Clal/Smal fragment of pSmal (Fig. 2). The resulting plasmid pSmaU3del (2792bp) was linearized by a Smal digest and ligated to a 3677bp Smal fragment of pBAGN (Fig. 3) to give the plasmid pBAGNU3del (6469bp). Deletion of the U3 region was confirmed by sequencing from the Clal-site into the U5-region using pSmaU3del as template.

A Clal/Spel (322bp) fragment containing the U3 deleted LTR was ligated to a Clal/Nhel fragment of pBAGNB (created by self ligation of a 3946pb fragment of pBAGN) to give the plasmid pCON6 (4097bp) (Fig. 3). This plasmid carrying a full U3-minus retroviral vector was used as a basis for further cloning.

According to the principle set forth above the following specific promoters have been inserted into the polylinker region or the modified BAG vector:

### Example 2

### Cloning of pMMTVgal

The Mouse Mammary Tumour Virus (MMTV) U3-Region (mtv-2) without the inverted repeats includes a region that confers responsiveness to glucocorticoid hormones and a region containing an element that directs expression to the mammary gland.

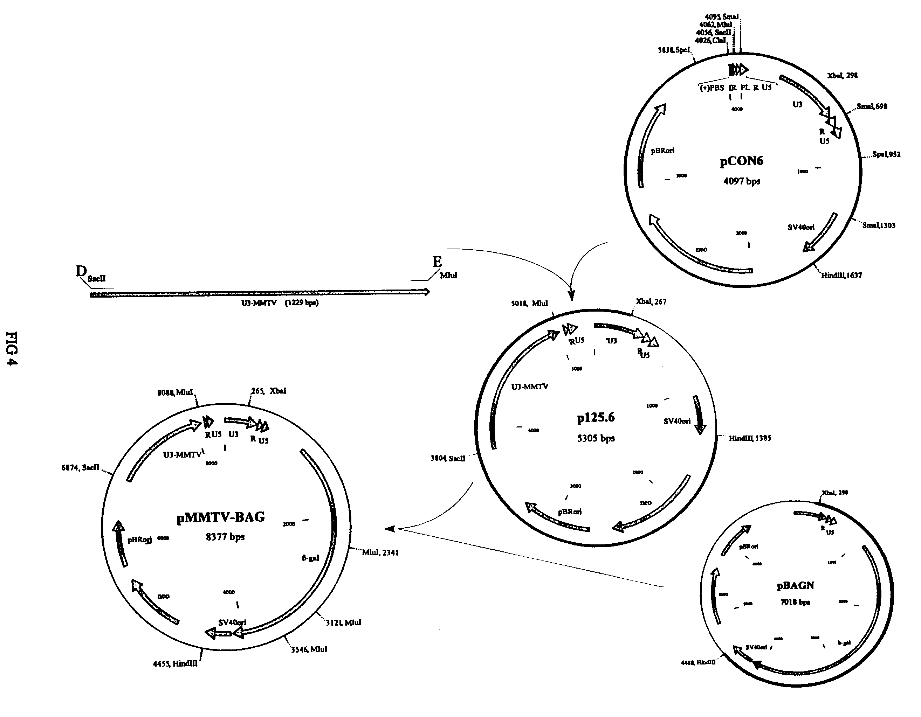

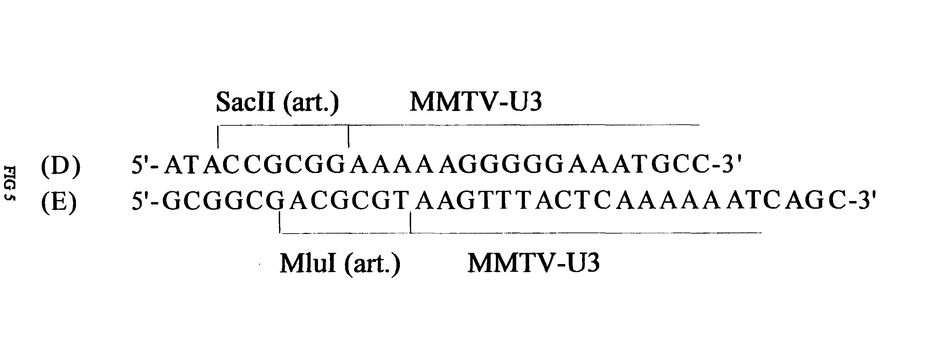

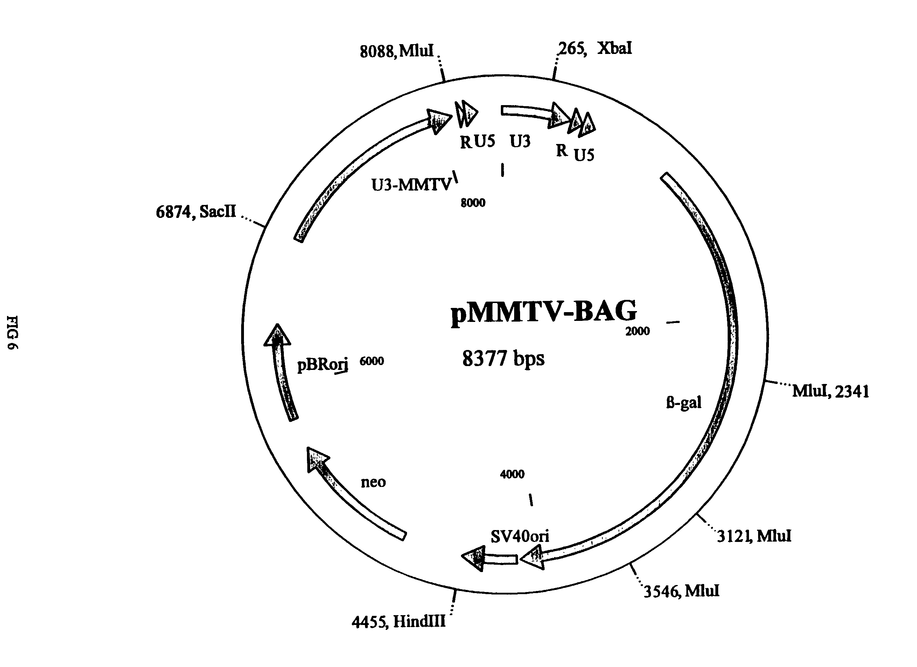

The U3 region of MMTV was amplified by PCR using the plasmid pBG102 (a plasmid containing the 3' LTR from mtv 2) as template with primers D and E.
The 3' end of primer D is complementary to the 5' end of the MMTV U3 region and carries a SacII site in its 5' extension (Fig. 5). The 3' end of primer E is complementary to the 3' end of the MMTV U3 region and has a Mlul site in its 5' extension (Fig. 5).
After 35 cycles of annealing at 49°C and extension at 72°C, a 1229bp product was obtained, digested with Sacll and Mlul and ligated to the SacII/Mlul digested vector pCON6. The resulting plasmid p125.6 (5305bp)(Fig. 4) was digested with Xbal and HindIII and the 4187 bp fragment ligated to the 4190bp fragment of pBAGN containing the β-galactosidase gene to give the plasmid pMMTV-BAG (8377bp)(Fig. 4) in which the β-galactosidase gene is under the transcriptional control of the MLV promoter after transfection, and under the MMTV promoter after infection (Fig. 6).

### Example 3

Cloning of the Whey Acidic Protein (WAP) promoter region encompassing the proximal 445 bp of the WAP promoter including the transcription initiation site.

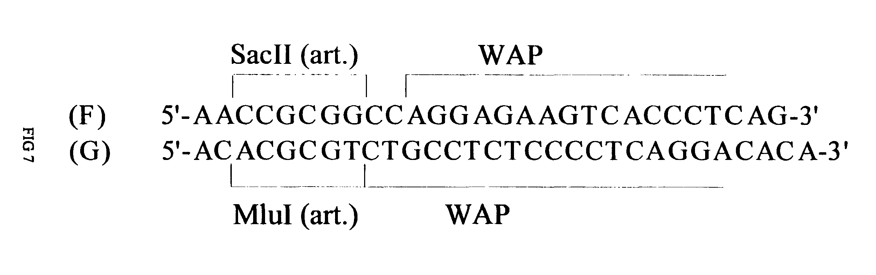

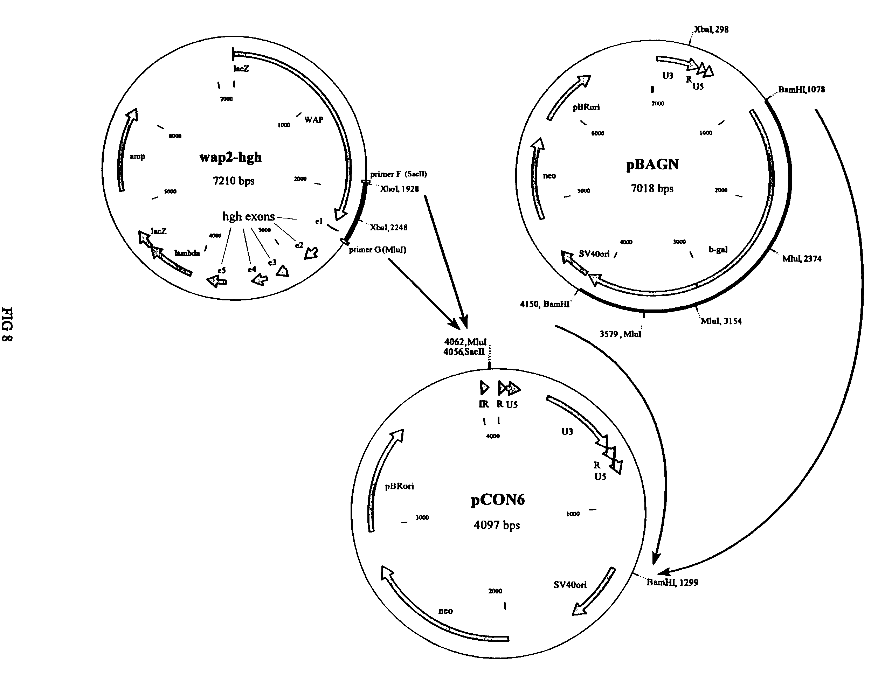

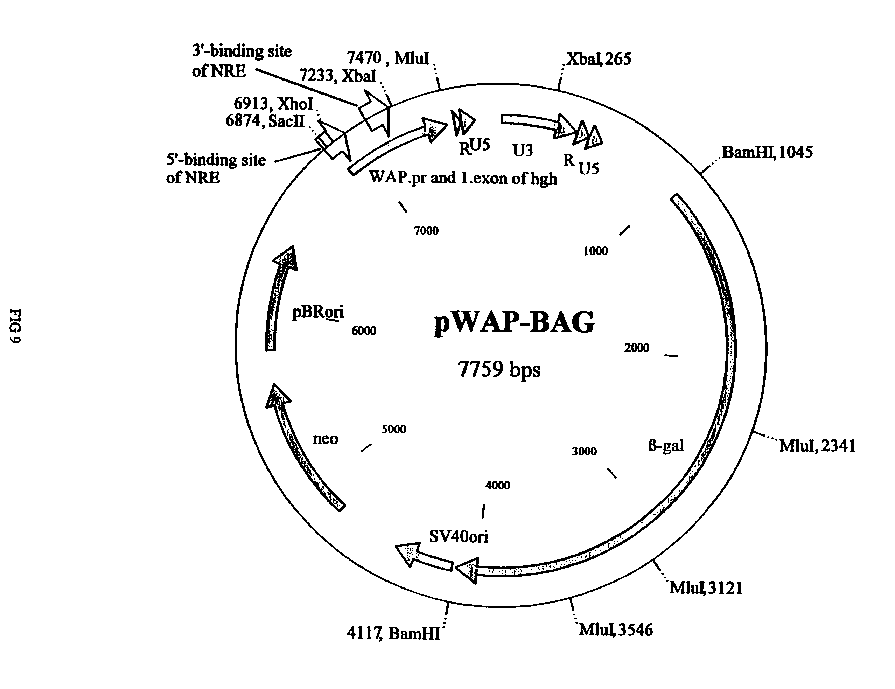

A plasmid, pWAPBAG containing the β-galactosidase gene under transcriptional control of the proximal 445 bp's of the WAP promoter was prepared by amplification of a sequence comprising the proximal 445 bp's of the WAP promoter and the first 143 bp's of the human growth hormone (HGH). The sequences were amplified from pWAP2-HGH (Günzburg et al., 1991) by PCR using primers F and G (Fig. 7). Both primers carried Sacll and Mlul recognition sites as terminal sequences. The amplified 606 bp product and pCON6 were digested with Sacll and Mlul and the 4094 bp fragment of the vector as well as the PCR product was ligated together to create pWAP.6. The β-galactosidase (β-gal) gene of E.coli was cloned into the resulting vector pWAP.6 (4687 bp) (Fig. 8): pWAP.6 as well as pBAGN were digested with BamHl and the linearised vector fragment as well as the 3072 bp β-gal fragment of pBAGN were ligated together. The resulting plasmid was pWAPBAG (Fig. 9), which is a ProCon vector in which the 3' 445 bp's containing the WAP-NRE, as well as the 5' 143 bp's of the HGH coding sequence, were inserted in place of the U3 region in the 3'LTR.

### Example 4

The control of the β-galactosidase gene expression under the transcriptional control of the WAP and MMTV promoters inserted into the polylinker has been validated by infection studies using the constructed MMTV and WAP retroviral vectors to infect various cells.

To produce retroviral vector particles, the MMTV and WAP ProCon vectors have been transfected into the packaging cell line PA317 (Miller and Buttimore, 1986). After selection for neomycin resistance, which is encoded by the vector, stable populations and clones of recombinant ProCon virus producing cells were obtained. Virus containing supernatant from these populations was used to infect explanted normal primary human mammary tissue obtained from reduction mammaplasties. Since it is known that the WAP and MMTV promoters are responsive to pregnancy hormones, the tissue was cultivated in the presence of 10⁻⁶ M dexamethasone, insulin (1µg/ml, EGF (5 ng/ml) and Cortisol (0.1 g/ml). The expression of the marker gene was determined by a quantitative β-gal assay which is based on the detection of β-galactosidase activity by chemiluminescence.

β-gal assay:

The levels of β-galactosidase activity was assayed using the Tropix kit acoording the manufacturers instructions. The cells were trypsinised,washed twice with PBS and lysed. The protein concentration was determined by a modified Lowry (BioRad DC protein assay). 5 and 10 µg of protein was used for the β-gal assay. The kit uses a substrate that is cleaved by the enzyme and thus the amount of enzyme activity is proportional to the amount of light produced. Expression was quantified by measuring chemiluminescence in a Berthold AutoLumat 953 (EG+G Berthold).
Detection of infected cells by histochemical staining was performed as outlined previously ([Cepko, 1989]). Briefly, the cells were washed with chilled PBS and then fixed with a 2% paraformaldehyde solution for 20 mins. After extensive washing with PBS, the cells were incubated in a solution containing the substrate X-gal (20mM K₃FeCN₆, 20mM K₄FeCN₆.3H₂O, 2mM MgCl₂ and 1mg/ml X-gal) for at least 2 hours at 37°C.

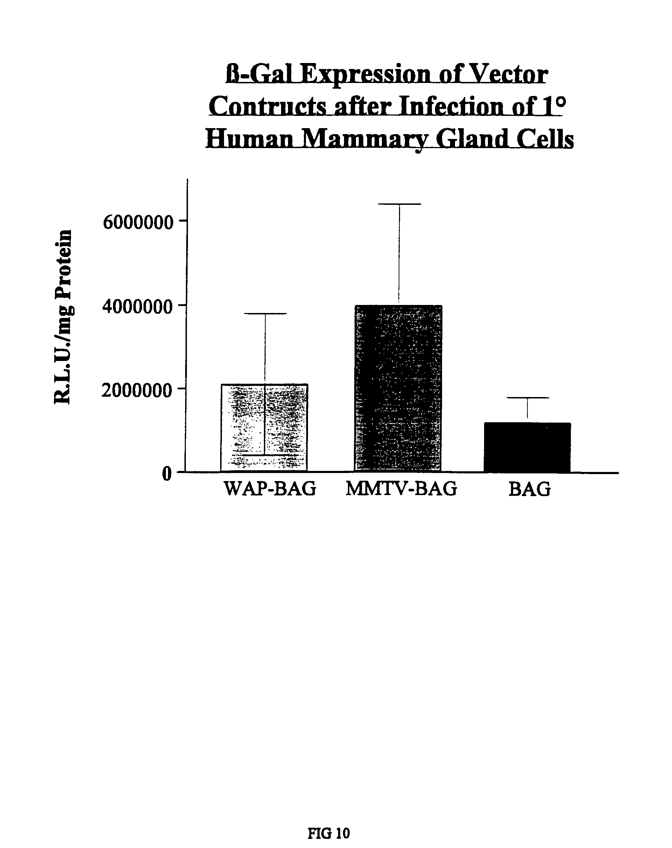

In all the experiments the original, non-tissue specific BAG-vector was used as a positive control. After infection of normal primary human mammary gland cells with BAG, WAPBAG and MMTVBAG, samples showed β-galactosidase expression (Fig. 10) in three independent experiments. It has thus been demonstrated for the first time that the WAP regulatory elements as well as the MMTV-U3 region can drive the expression of a gene within a MLV retroviral vector in primary human mammary gland cells.

### Example 5

Cytochrome P450 catalyses the hydroxylation of the commonly used cancer prodrugs cyclophosphamide (CPA) and ifosfamide to their active toxic forms. Normally the expression of the patient's endogenous cytochrome P450 gene is limited to the liver, and anti-tumor effects of systemically applied CPA's depend upon the subsequent systemic distribution of toxic drug metabolites from the liver. This has led to toxicity problems since the activated drug not only affects the tumor but also affects other normal patient tissues such as bone marrow and kidney.

A therapeutic approach, where the cytochrome P450 gene is selectively introduced directly into tumor cells, and overexpressed in these cells, would circumvent this problem. Toxic metabolites produced from the transduced tumor cells affect surrounding non-transduced tumor cells in a concentration gradient dependent manner. An additional advantage of the cytochrome P-450/CPA system is the lack of dependency upon cell replication for cytotoxic effects on the surrounding cells. This is because one of the active metabolites generated causes interstrand crosslinks regardless of the cell cycle phase. Later on, during DNA synthesis, these interstrand crosslinks result in cell death.

Construction of a retroviral vector carrying the rat cytochrome P450 gene under control of the WAP regulatory sequence:
To yield the rat cytochrome P450 2B1 gene cells of the rat hepatoma cell line HTC were lysed with solution D (4M guanidium thiocyanate, 25mM sodium citrate pH7, 0.5%N-laurylsarcosine sodium, 0.1M 2-mercaptoethanol) and total RNA extracted by adding 1/15 volume of 3M sodium acetate, in the same volume of watersaturated phenol and 1/5 volume of chloroform/isoamyalcolhol (49:1) were added and the whole mixture mixed vigorously. After 15 min on ice the extract was centrifuged 20min at 4°C at 10.000g. The RNA in the Aqueous phase was precipitated with one volume of isopropanol for 30min at-20°C and centrifuged at 10.000g at 4°C. The pellet was washed in 70% ethanol and left at room temperature for 15 min. After 5 min centrifugation at 4°C and 10.000g the pellet was dried in a vacuum dryer and redissolved in 0.5% SDS solution.

The extracted RNA is reverse transcribed using the protocol for cDNA synthesis (Pharmacia). The resulting cDNA is used as template for a PCR. The primers are designed so that they contained a BamHI restriction site (underlined) in the lefthand primer (e.g.5'-AAGCCGGATCCCTGGAGAGCATGCAC-3') and a BamHI site (underlined) in the righthand primer ( e.g. 5'-CGATTAGGATCCCTGCCTCA-3'). Both primers have additional bases at the 5'-end for higher efficiency of cleavage by the relevant restriction enzyme. The 1562 bp-product is digested with BamHI and the fragment obtained is containing the gene for P450 is ligated into the BamHI digested plasmid pWAP.6 (example 3).

One day before lipofection 3x10⁵ retroviral packaging cells are seeded into 6cm petri or culture dishes. On the day of infection 2µg of the vector encoding cytochrome P450 under transcriptional control of the WAP regulatory sequence are mixed with 100µl serum free media. In parallel 15µl of Lipofectamine (Gibco BRL) is mixed with 100µl serumfree media. The plasmid containing solution is added to the Lipofectamine-mix and incubated for 45min. After 35 min the cell are washed once with 2ml serum free media. 800µl of serum free media were added to the lipofection-mix and the resulting 1 ml is put onto the prepared cells. After 6 hours 1ml Dulbecco's modified Eagles medium containing 10% FCS is added. The next day the cells are trypsinized and 1:10 diluted and seeded on a 100mm dish. After 24h the media is replaced with medium containing the neomycin analog G418. Single cell clones or cell populations are isolated and analysed for expression of cytochrome P450.

### Encapsulation of cells and implantation of encapsulated cells

The retroviral vector producing packaging cells are suspended in 1ml of 0.5-50%, but preferably 2-5%, anionic polymer (e.g. sodium cellulose sulphate) solution which also contains 5% fetal calf serum. This suspension is then dropped by a dispensing system (e.g. A-jet system or piezoelectric system) into a precipitation bath containing a stirred 0.5%-50% polymeric polycation (e.g. polydimethyldiallylammonium). The capsule formation occurs within milliseconds and the capsules containing cells are kept in the precipitation bath for 30 seconds to 5 minuntes and then washed. The rapidity of this method ensures that the cells are not unduly stressed during the whole procedure (Stange et al., 1993).

The capsules producing viral particles is implanted in or around the mammary tumor in capsules thereby ensuring continuous release of virus.

Alternatively the virus could be introduced by multible direct injections into the mammary tumour. Systemic or local administration of cyclophosphamide or ifosfamide will result in local conversion of these compounds to their toxic forms leading to ablation of tumour cells.

### References

Anderson, W.F. 1992. Human gene therapy. Science 256: 808-813.

Cone, R.D. and Mulligan R.C., 1984 Proc. Natl. Acad. Sci. USA 81, 6349-6353.

Cosset F.L., Takeuchi, Y., Batini, J.L., Weiss, R.A. and Collins; M. K. L., 1995, J. Virol. 69, 7430-7436.

Danos, O., and Mulligan, R.C., 1988, Proc. Natl. Acad. Sci. USA 85, 6460-6464.

Dautzenberg, H., et al., 1993, Biomat., Art. Cells & Immob. Biotech., 21(3), 399-405.

Graessmann et al., 1983, Meth. Enzymology 101, 482-492.

Graham et al., 1977. J.Gen. Virol. 36, 59.

Graham et al., 1973, Virol. 52, 456-467.

Günzburg, W.H., Salmons, B., Zmmermann, B., Müller, M., Brem, G. 1991, Molcular Endocrinology 5, 123-133.

Günzburg W.H., and Salmons, B., 1995, Mol. Med. Today 1, 410-417.

Hennighausen, L. 1992. J. Cell. Biochem. 49:325-332.

Kolb, A.F., Günzburg, W.H., Albang, R., Brem, G., Erfle, V. and Salmons, B. 1994. J. Cell. Biochem. 56:245-261.

Kolb, A.F., Günzburg, W.H., Albang, R., Brem, G., Erfle, V. and Salmons, B. 1995. Biochem. Biophys. Res. Commun. 217, 1045-1052.

Mann, M., Mulligan R.C. and Buttimore, D., 1983. Cell 33, 153-159.

Markowitz, D., S. Goff, and A. Bank. 1988a. J. Virol. 62: 1120-1124.

Markowitz, D., S. Goff, and A. Bank. 1988b. Virology 167, 400-406.

Merten, O. W. et al., Cytotechnology: 121-130, 1991.

Miller A.B. and Bulbrook, R.D. 1986. Int. J. Cancer. 37:173-177.

Miller A.D. and Buttimore, C. 1986. Mol. Cell. Biol. 6,2895-2902.

Miller, A.D. and G.J. Rossman. 1989. Biotechniques 7: 980-990.

Neumann et al., EMBO J. 1, 841-845 (1982).

Pear, W.S. Nolan, G.P., Scott, M.L. and Buttimore D., 1993. Proc Natl. Acad. Sci. USA 90, 8392-8396.

Perbal, B. 1984. A Practical Guide to Molecular Cloning, John Wiley & Sons.

Price, J., D. Turner, and C. Cepko. 1987. Proc. Natl. Acad. Sci. USA 84: 156-160.

Salmons, B., Groner., B. Calberg Baca, C.M. and Ponta H, 1985. Virology 144, 101-114.

Salmons, B. and W.H. Günzburg. 1993. Human Gene Therapy 4: 129-141.

Sambrook, J., E.F. Fritsch and T. Maniatis. 1989. Molecular Cloning. Cold Spring Harbor Laboratory Press, New York, USA.

Schaffner, Proc. Natl. Acad. Sci. USA 77, 2163-2167 (1980).

Stange, J. et al., 1993, Biomat., Art. Cells & Immob. Biotech., 21(3), 343-352.

Straubinger et al., 1983, Methods in Enzymology 101, 512-527.

Varmus, H. 1988. Retroviruses. Science 240: 1427-1435.

Wigler et al., 1979, Cell 777-785.

## Claims

1. A DNA construct comprising at least one therapeutic gene under transcriptional control of the WAP or MMTV regulatory sequences for the treatment of disorders or diseases of human mammary cells, including human mammary carcinoma.

2. A DNA construct according to claim 1 wherein the regulatory sequence comprises the proximal 445 bp of the WAP promoter including the transcription initiation site.

3. A DNA construct according to claims 1 to 2 wherein the regulatory sequence contain the 320 bp Xhol/Xbal fragment of the WAP promoter region.

4. A DNA construct according to claim 1 wherein the regulatory sequence is the U3 region of MMTV.

5. A DNA construct according to claims 1 and 4 wherein the regulatory sequence contain the 0.6 Kb Pstl MMTV promoter fragment .

6. A DNA construct according to claims 1 to 5 which is a recombinant vector selected from viral and plasmid vectors.

7. A recombinant vector according to claim 6 wherein said viral vector is selected from RNA and DNA viral vectors and said plasmid vector is selected from eucaryotic expression vectors.

8. A recombinant vector according to claim 7 wherein said viral vector is a retroviral vector, a recombinant adenovirus vector, a recombinant adeno-associated virus vector or a recombinant herpes virus vector.

9. A recombinant vector according to claim 8 wherein the retroviral vector comprises a 5'LTR region of the structure U3-R-U5; at least one coding sequence coding for a therapeutic gene; and a 3' LTR region comprising a completely or partially deleted U3 region wherein said deleted region has been replaced by a polylinker containing the WAP or MMTV regulatory sequences followed by the R and U5 region, said therapeutic gene being under transcriptional control of the WAP or MMTV regulatory sequences.

10. A construct according to claims 1 to 9 wherein the therapeutic gene is selected from anti-tumor genes and cytokine genes for the treatment of human mammary carcinoma.

11. A construct according to claim 10, wherein said therapeutic gene is selected from the group consisting of genes which code for proteins such as Herpes Simplex Virus thymidine kinase, cytosine deaminase, guanine phosphoribosyl transferase (gpt), cytochrome P 450, cell cycle regulatory genes which code for proteins such as SDI, tumor supressor genes which code for proteins such as p53, antiproliferation genes which codes for proteins such as melittin, cecropin or cytokines such as IL-2.

12. A recombinant retroviral particle produced by culturing a packaging cell line harbouring a retroviral vector construct according to claims 8 to 9 and one or more constructs coding for the proteins required for the genome of said retroviral vector to be packaged, for the treatment of disorders or diseases of human mammary cells, including human mammary carcinoma.

13. A retroviral provirus carrying a construct comprising at least one therapeutic gene under transcriptional control of the WAP or MMTV regulatory sequences integrated in the human genome.

14. A retroviral provirus according to claim 13 comprising a 5'LTR region comprising a completely or partially deleted U3 region wherein said deleted region has been replaced by a polylinker containing the WAP or MMTV regulatory sequences followed by the R and U5 region; at least one coding sequence coding for a therapeutic gene; and a 3' LTR region comprising a completely or partially deleted U3 region wherein said deleted region has been replaced by a polylinker containing the WAP or MMTV regulatory sequences followed by the R and U5 region, said therapeutic gene being under transcriptional control of the WAP or MMTV regulatory sequences.

15. A cell line containing a construct according to claims 1 to 11 for the treatment of disorders or diseases of human mammary cells, including human mammary carcinoma.

16. A cell line according to claim 15 which is a packaging cell line harbouring a retroviral vector construct according to claims 8 to 9 and one or more constructs coding for the proteins required for the genome of said retroviral vector to be packaged.

17. A human cell containing a retroviral provirus according to claims 13 to 14.

18. Encapsulated cells comprising a core containing cells according to claims 15 to 17 and a porous capsule wall surrounding said core, said porous capsule wall being permeable to the therapeutic polypeptide or the viral particles produced by said cells for the treatment of disorders or diseases of human mammary cells, including human mammary carcinoma.

19. Encapsulated cells according to claim 18 wherein said porous capsule wall consists of a polyelectrolyte complex formed from counter charged polyelectrolytes.

20. The use of a construct according to claims 1 to 11 for the preparation of a medicament for the treatment of disorders or diseases of human mammary cells, including human mammary carcinoma.

21. The use of a recombinant viral particle according to claims 12 for the manufacture of a medicament for the treatment of disorders or diseases of human mammary cells, including human mammary carcinoma.

22. The use of cells according to claims 15 to 17 for the manufacture of a medicament for the treatment of a disorder or disease of human mammary cells, including human mammary carcinoma.

23. A pharmaceutical composition for the treatment of disorders or diseases of human mammary cells, including human mammary carcinoma comprising a
DNA construct according to claims 1 to 11 and a pharmaceutically acceptable carrier or diluent.

24. A pharmaceutical composition for the treatment of disorders or diseases of human mammary cells, including human mammary carcinoma comprising a
a recombinant retroviral particle according to claim 12 and a pharmaceutically acceptable carrier or diluent.

25. A pharmaceutical composition for the treatment of disorders or diseases of human mammary cells, including human mammary carcinoma comprising a
a cell line according to claims 15 to 17 and a pharmaceutically acceptable carrier or diluent.

26. The use of the WAP or MMTV regulatory sequences for the in vitro-expression of linked therapeutic genes in human mammary cells, including human mammary carcinoma cells.

27. The use according to claims 26 wherein the regulatory sequence comprises the proximal 445 bp of the WAP promoter including the transcription initiation site.

28. The use according to claims 26 to 27 wherein the regulatory sequence contain the 320 bp XhoI/XbaI fragment of the WAP promoter region.

29. The use according to claim 26 wherein the regulatory sequence is the U3 region of MMTV.

30. The use according to claims 26 and 29 wherein the regulatory sequence contain the 0.6 Kb Pstl MMTV promoter fragment.

31. The use according to claims 26 to 30 wherein the therapeutic gene is selected from anti-tumor genes and cytokine genes.

32. The use according to claim 31, wherein said therapeutic gene is selected from the group consisting of genes which code for proteins such as Herpes Simplex Virus thymidine kinase, cytosine deaminase, guanine phosphoribosyl transferase (gpt), cytochrome P 450, cell cycle regulatory genes which code for proteins such as SDI, tumor supressor genes which code for proteins such as p53, antiproliferation genes which codes for proteins such as melittin, cecropin or cytokines such as IL-2.

33. The use according to claims 26 to 32 wherein the therapeutic gene under transcriptional control of the WAP or MMTV regulatory sequences form part of a recombinant vector selected from viral and plasmid vectors.

34. The use according to claim 33 wherein said viral vector is selected from RNA and DNA viral vectors and said plasmid vector is selected from eucaryotic expression vectors.

35. The use according to claim 34 wherein said viral vector is a retroviral vector.

36. The use according to claim 35 wherein the retroviral vector comprises a 5'LTR region of the structure U3-R-U5; at least one coding sequence coding for a therapeutic gene; and a 3' LTR region comprising a completely or partially deleted U3 region wherein said deleted region has been replaced by a polylinker containing the WAP or MMTV regulatory sequences followed by the R and U5 region, said therapeutic gene being under transcriptional control of the WAP or MMTV regulatory sequences.

## Patentansprüche

1. DNA-Konstrukt, enthaltend wenigstens ein therapeutisches Gen unter der transkriptionellen Kontrolle der WAP oder MMTV regulatorischen Sequenzen zur Behandlung von Störungen oder Erkrankungen von humanen Mamma-Zellen, einschließlich des humanen Mammakarzinomes.

2. DNA-Konstrukt nach Anspruch 1, wobei die regulatorische Sequenz die proximalen 445 Bp des WAP-Promotors einschließlich der Transkriptionsinitiationsstelle enthält.

3. DNA-Konstrukt nach den Ansprüchen 1 bis 2, wobei die regulatorische Sequenz das 320 Bp Xhol/Xbal Fragment der WAP-Promotorregion enthält.

4. DNA-Konstrukt nach Anspruch 1, wobei die regulatorische Sequenz die U3-Region von MMTV ist.

5. DNA-Konstrukt nach den Ansprüchen 1 und 4, wobei die regulatorische Sequenz das 0,6 kb PstI MMTV-Promotorfragment enthält.

6. DNA-Konstrukt nach den Ansprüchen 1 bis 5, bei dem es sich um einen rekombinanten Vektor, ausgewählt aus viralen und Plasmid-Vektoren, handelt.

7. Rekombinanter Vektor nach Anspruch 6, wobei der virale Vektor aus RNA und DNA viralen Vektoren und der Plasmidvektor aus eukaryontischen Expressionsvektoren ausgewählt ist.

8. Rekombinanter Vektor nach Anspruch 7, wobei der virale Vektor ein retroviraler Vektor, ein rekombinanter Adenovirusvektor, ein rekombinanter Adeno-Assoziierter-Virusvektor oder ein rekombinanter Herpesvirusvektor ist.

9. Rekombinanter Vektor nach Anspruch 8, wobei der retrovirale Vektor eine 5'LTR Region der Struktur U3-R-U5, wenigstens eine kodierende Sequenz, die für ein therapeutisches Gen kodiert, und eine 3'LTR Region, die einen vollständig oder teilweise deletierten U3-Bereich, gefolgt von dem R-Bereich und dem U5-Bereich, umfaßt, wobei der deletierte Bereich durch einen Polylinker ersetzt wurde, der die WAP oder MMTV regulatorischen Sequenzen enthält, umfaßt, und wobei das therapeutische Gen unter der transkriptionellen Kontrolle der WAP oder MMTV regulatorischen Sequenzen steht.

10. Konstrukt nach den Ansprüchen 1 bis 9, wobei das therapeutische Gen ausgewählt ist aus anti-Tumorgenen und Zytokingenen, zur Behandlung des humanen Mamma-Karzinoms.

11. Konstrukt nach Anspruch 10, wobei das therapeutische Gen ausgewählt ist aus der Gruppe bestehend aus Genen, die für Proteine, wie Herpes-simplexVirus-Thymidinkinase, Cytosindesaminase, Guaninphosphoribosyltransferase (gpt), Cytochrom P 450 kodieren, Zellzyklus-regulatorischen Genen, die für Proteine wie SDI kodieren, Tumorsuppressorgenen, die für Proteine wie p53 kodieren, Antiproliferationsgenen, die für Proteine wie Melittin, Cecropin kodieren, oder Cytokinen wie IL-2.

12. Rekombinantes retrovirales Partikel, hergestellt durch Kultivieren einer Verpackungszellinie, die ein retrovirales Vektorkonstrukt nach den Ansprüchen 8 bis 9 und ein oder mehrere Konstrukt(e), die für die Proteine kodieren, die benötigt werden, damit das Genom des retroviralen Vektors verpackt wird, enthält, zur Behandlung von Störungen oder Erkrankungen humaner Mammazellen, einschließlich des humanen Mammakarzinoms.

13. Retrovirales Provirus, das ein Konstrukt enthält, das wenigstens ein therapeutisches Gen unter der transkriptionellen Kontrolle der WAP oder MMTV regulatorischen Sequenzen enthält, und das in das humane Genom integriert ist.

14. Retrovirales Provirus nach Anspruch 13, umfassend einen 5'LTR Bereich, umfassend einen vollständig oder teilweise deletierten U3-Bereich, gefolgt von dem R- und U5-Bereich, wobei der deletierte Bereich durch einen Polylinker, der die WAP oder MMTV regulatorischen Sequenzen enthält, ersetzt wurde; wenigstens eine kodierende Sequenz, die für ein therapeutisches Gen kodiert; und einen 3'LTR Bereich, umfassend einen vollständig oder teilweise deletierten U3-Bereich, gefolgt von dem R- und US-Bereich, wobei der deletierte Bereich durch einen Polylinker, der die WAP oder MMTV regulatorischen Sequenzen enthält, ersetzt wurde, wobei das therapeutische Gen unter der transkriptionellen Kontrolle der WAP und MMTV regulatorischen Sequenzen steht.

15. Zellinie enthaltend ein Konstrukt nach den Ansprüchen 1 bis 11 zur Behandlung von Störungen oder Erkrankungen von humanen Mamma-Zellen, einschließlich des humanen Mamma-Karzinoms.

16. Zellinie nach Anspruch 15, nämlich eine Verpackungszellinie, die ein retrovirales Vektorkonstrukt nach den Ansprüchen 8 bis 9 und ein oder mehrere Konstrukt(e), die für die.Proteine kodieren, die benötigt werden, damit der retrovirale Vektor verpackt wird, enthält.

17. Humane Zellinie, die ein retrovirales Provirus nach den Ansprüchen 13 bis 14 enthält.

18. In einer Kapsel eingeschlossene Zellen, umfassend einen Kern, der Zellen nach den Ansprüchen 15 bis 17 enthält, und eine poröse Kapselwand, die den Kern umgibt, wobei die poröse Kapselwand für das therapeutische Polypeptid oder die viralen Partikel, das/die von den Zellen produziert wird/werden, permeabel ist, zur Behandlung von Störungen oder Erkrankungen von humanen Mamma-Zellen, einschließlich des humanen Mamma-Karzinoms.

19. In einer Kapsel eingeschlossene Zellen nach Anspruch 18, wobei die poröse Kapselwand aus einem Polyelektrolyt-Komplex besteht, der von Polyelektrolyten entgegengesetzter Ladung gebildet wird.

20. Verwendung eines Konstruktes nach den Ansprüchen 1 bis 11 zur Herstellung eines Medikamentes zur Behandlung von Störungen oder Erkrankungen von humanen Mamma-Zellen, einschließlich des humanen Mamma-Karzinoms.

21. Verwendung eines rekombinanten viralen Partikels nach Anspruch 12 zur Herstellung eines Medikaments zur Behandlung von Störungen oder Erkrankungen von humanen Mamma-Zellen, einschließlich des humanen Mamma-Karzinoms.

22. Verwendung von Zellen nach den Ansprüchen 15 bis 17 zur Herstellung eines Medikaments zur Behandlung einer Störung oder Erkrankung humaner Mamma-Zellen, einschließlich des humanen Mamma-Karzinoms.

23. Pharmazeutische Zusammensetzung zur Behandlung von Störungen oder Erkrankungen von humanen Mamma-Zellen, einschließlich des humanen Mamma-Karzinoms, enthaltend ein DNA-Konstrukt nach den Ansprüchen 1 bis 11 und einen pharmazeutisch annehmbaren Träger oder ein pharmazeutisch annehmbares Verdünnungsmittel.

24. Pharmazeutische Zusammensetzung zur Behandlung von Störungen oder Erkrankungen von humanen Mamma-Zellen, einschließlich des humanen Mamma-Karzinoms, enthaltend ein rekombinantes retrovirales Partikel nach Anspruch 12 und einen pharmazeutisch annehmbaren Träger oder ein pharmazeutisch annehmbares Verdünnungsmittel.

25. Pharmazeutische Zusammensetzung zur Behandlung von Störungen oder Erkrankungen von humanen Mamma-Zellen, einschließlich des humanen Mamma-Karzinoms, enthaltend eine Zellinie nach den Ansprüchen 15 bis 17 und einen pharmazeutisch annehmbaren Träger oder ein pharmazeutisch annehmbares Verdünnungsmittel.

26. Verwendung der WAP oder MMTV regulatorischen Sequenzen für die in vitro- Expression damit verbundener therapeutischer Gene in humanen Mammazellen, einschließlich humaner Mammakarzinomzellen.

27. Verwendung nach Anspruch 26, wobei die regulatorische Sequenz die proximalen 445 Bp des WAP-Promotors einschließlich der Transkriptionsinitiationsstelle umfaßt.

28. Verwendung nach den Ansprüchen 26 bis 27, wobei die regulatorische Sequenz das 320 Bp Xhol/Xbal-Fragment der WAP-Promotor-Region enthält.

29. Verwendung nach Anspruch 26, wobei die regulatorische Sequenz der U3-Bereich von MMTV ist.

30. Verwendung nach den Ansprüchen 26 und 29, wobei die regulatorische Sequenz das 0,6 kb Pstl MMTV-Promotorfragment enthält.

31. Verwendung nach den Ansprüchen 26 bis 30, wobei das therapeutische Gen ausgewählt ist aus Antitumorgenen und Zytokingenen.

32. Verwendung nach Anspruch 31, wobei das therapeutische Gen ausgewählt ist aus der Gruppe bestehend aus Genen, die für Proteine, wie Herpessimplex-Virus-Thymidinkinase, Cytosindesaminase, Guaninphosphoribosyltransferase (gpt), Cytochrom P 450 kodieren, Zellzyklus-regulatorischen Genen, die für Proteine wie SDI kodieren, Tumorsuppressorgenen, die für Proteine wie p53 kodieren, Antiproliferationsgenen, die für Proteine wie Melittin, Cecropin kodieren, oder Cytokinen wie IL-2.

33. Verwendung nach den Ansprüchen 26 bis 32, wobei das therapeutische Gen unter transkriptioneller Kontrolle der WAP oder MMTV regulatorischen Sequenzen Teil eines rekombinanten Vektors, ausgewählt aus viralen und Plasmid-Vektoren, ist.

34. Verwendung nach Anspruch 33, wobei der virale Vektor ausgewählt ist aus RNA und DNA viralen Vektoren und der Plasmidvektor ausgewählt ist aus eukaryontischen Expressionsvektoren.

35. Verwendung nach Anspruch 34, wobei der virale Vektor ein retroviraler Vektor ist.

36. Verwendung nach Anspruch 35, wobei der retrovirale Vektor eine 5'LTR Region der Struktur U3-R-U5, wenigstens eine kodierende Sequenz, die für ein therapeutisches Gen kodiert, und eine 3'LTR Region, die einen vollständig oder teilweise deletierten U3-Bereich, gefolgt von dem R-Bereich und dem U5-Bereich, umfaßt, wobei der deletierte Bereich durch einen Polylinker ersetzt wurde, der die WAP oder MMTV regulatorischen Sequenzen enthält, umfaßt, und wobei das therapeutische Gen unter der transkriptionellen Kontrolle der WAP oder MMTV regulatorischen Sequenzen steht.

## Revendications

1. Structure d'ADN comprenant au moins un gène thérapeutique sous contrôle transcriptionnel des séquences régulatrices de la protéine acide du lactosérum ou du virus de la tumeur mammaire de la souris, pour le traitement de troubles ou maladies de cellules mammaires humaines, notamment le cancer mammaire humain.

2. Structure d'ADN selon la revendication 1, dans laquelle la séquence régulatrice comprend les 445 pb proximales du promoteur de la protéine acide du lactosérum, comportant le site d'initiation de la transcription.

3. Structure d'ADN selon les revendications 1 à 2, dans laquelle la séquence régulatrice contient le fragment Xhol/Xbal à 320 pb de la région du promoteur de la protéine acide du lactosérum.

4. Structure d'ADN selon la revendication 1, dans laquelle la séquence régulatrice est la région U3 du virus de la tumeur mammaire de la souris.

5. Structure d'ADN selon les revendications 1 et 4, dans laquelle la séquence régulatrice contient le fragment PstI à 0,6 Kb du promoteur du virus de la tumeur mammaire de la souris.

6. Structure d'ADN selon les revendications 1 à 5, qui est un vecteur recombiné sélectionné parmi des vecteurs viraux et plasmidiques.

7. Vecteur recômbiné selon la revendication 6, dans lequel ledit vecteur viral est sélectionné parmi des vecteurs viraux d'ARN et d'ADN et ledit vecteur plasmidique est sélectionné parmi des vecteurs d'expression eucaryotique.

8. Vecteur recombiné selon la revendication 7, dans lequel ledit vecteur viral est un vecteur rétroviral, un vecteur recombiné d'adénovirus, un vecteur recombiné du virus adéno-associé, ou un vecteur recombiné du virus de l'herpès.

9. Vecteur recombiné selon la revendication 8, dans lequel le vecteur rétroviral comprend une région 5'LTR de la structure U3-R-U5 ; au moins une séquence codante codant pour un gène thérapeutique ; et une région 3'LTR comprenant une région U3 complètement ou partiellement délétée, dans laquelle ladite région délétée a été remplacée par un lieur multisite contenant les séquences régulatrices de la protéine acide du lactosérum ou du virus de la tumeur mammaire de la souris suivies par les régions R et U5, ledit gène thérapeutique étant sous contrôle transcriptionnel des séquences régulatrices de la protéine acide du lactosérum ou du virus de la tumeur mammaire de la souris.

10. Structure selon les revendications 1 à 9, dans laquelle le gène thérapeutique est sélectionné parmi des gènes anti-tumoraux et des gènes de cytokines pour le traitement du cancer mammaire humain.

11. Structure selon la revendication 10, dans laquelle ledit gène thérapeutique est sélectionné parmi le groupe constitué de gènes qui codent pour des protéines telles que la thymidine-kinase du virus de l'herpès, la cytosine-déaminase, la guanine-phosphoribosyl-transférase (gpt), le cytochrome P 450, de gènes régulateurs de cycle de la cellule qui codent pour des protéines tels que le gène SDI, de gènes suppresseurs de tumeur qui codent pour des protéines tels que le gène p53, les gènes d'antiprolifération qui codent pour des protéines telles que la melittine, la cécropine ou des cytokines telles que l'IL-2.

12. Particule rétrovirale recombinée produite par la mise en culture d'une lignée cellulaire d'empaquetage abritant une structure de vecteur rétroviral selon les revendications 8 à 9 et une ou plusieurs structures codant pour les protéines nécessaires pour le génome dudit vecteur rétroviral à empaqueter, pour le traitement de troubles ou maladies des cellules mammaires humaines, notamment le cancer mammaire humain.

13. Provirus rétroviral transportant une structure comprenant au moins un gène thérapeutique sous contrôle transcriptionnel des séquences régulatrices de la protéine acide du lactosérum ou du virus de la tumeur mammaire de la souris intégrées dans le génome humain.

14. Provirus rétroviral selon la revendication 13 comprenant une région 5'LTR comprenant une région U3 complètement ou partiellement délétée, dans lequel ladite région délétée a été remplacée par un lieur multisite contenant les séquences régulatrices de la protéine acide du lactosérum ou du virus de la tumeur mammaire de la souris suivies par les régions R et U5 ; au moins une séquence codante codant pour le gène thérapeutique ; et une région 3'LTR comprenant une région U3 complètement ou partiellement délétée dans lequel ladite région délétée a été remplacée par un lieur multisite contenant les séquences régulatrices de la protéine acide du lactosérum ou du virus de la tumeur mammaire de la souris suivies par les régions R et U5, ledit gène thérapeutique étant sous contrôle transcriptionnel des séquences régulatrices de la protéine acide du lactosérum ou du virus de la tumeur mammaire de la souris.

15. Lignée cellulaire contenant une structure selon les revendications 1 à 11 pour le traitement de troubles ou maladies des cellules mammaires humaines, notamment le cancer mammaire humain.

16. Lignée cellulaire selon la revendication 15 qui est une lignée cellulaire d'empaquetage abritant une structure de vecteur rétroviral selon les revendications 8 à 9 et une ou plusieurs structures codant pour les protéines nécessaires pour le génome dudit vecteur rétroviral à empaqueter.

17. Cellule humaine contenant un provirus rétroviral selon les revendications 13 à 14.

18. Cellules encapsulées comprenant un noyau contenant des cellules selon les revendications 15 à 17 et une paroi de capsule poreuse entourant ledit noyau, ladite paroi de capsule poreuse étant perméable au polypeptide thérapeutique ou aux particules virales produites par lesdites cellules pour le traitement de troubles ou maladies des cellules mammaires humaines, notamment le cancer mammaire humain.

19. Cellules encapsulées selon la revendication 18, dans lesquelles ladite paroi de capsule poreuse est constituée d'un complexe polyélectrolytique formé à partir de polyélectrolytes chargés de façon inverse.

20. Utilisation d'une structure selon les revendications 1 à 11 pour la préparation d'un médicament pour le traitement de troubles ou maladies des cellules mammaires humaines, notamment le cancer mammaire humain.

21. Utilisation d'une particule virale recombinée selon la revendication 12 pour la fabrication d'un médicament pour le traitement de troubles ou maladies des cellules mammaires humaines, notamment le cancer mammaire humain.

22. Utilisation de cellules selon les revendications 15 à 17 pour la fabrication d'un médicament pour le traitement d'un trouble ou d'une maladie des cellules mammaires humaines, notamment le cancer mammaire humain.

23. Composition pharmaceutique pour le traitement de troubles ou maladies des cellules mammaires humaines, notamment le cancer mammaire humain, comprenant une structure d'ADN selon les revendications 1 à 11 et un véhicule ou diluant pharmaceutiquement acceptable.

24. Composition pharmaceutique pour le traitement de troubles ou maladies des cellules mammaires humaines, notamment le cancer mammaire humain, comprenant une particule rétrovirale recombinée selon la revendication 12 et un véhicule ou diluant pharmaceutiquement acceptable.

25. Composition pharmaceutique pour le traitement de troubles ou maladies des cellules mammaires humaines, notamment le cancer mammaire humain, comprenant une lignée cellulaire selon les revendications 15 à 17 et un véhicule ou diluant pharmaceutiquement acceptable.

26. Utilisation des séquences régulatrices de la protéine acide du lactosérum ou du virus de la tumeur mammaire de la souris pour l'expression in vitro de gènes thérapeutiques liés dans des cellules mammaires humaines, notamment des cellules cancéreuses mammaires humaines.

27. Utilisation selon la revendication 26, dans laquelle la séquence régulatrice comprend les 445 pb proximales du promoteur de la protéine acide du lactosérum, notamment le site d'initiation de la transcription.

28. Utilisation selon les revendications 26 à 27, dans laquelle la séquence régulatrice contient le fragment Xhol/Xbal à 320 pb de la région du promoteur de la protéine acide du lactosérum.

29. Utilisation selon la revendication 26, dans laquelle la séquence régulatrice est la région U3 du virus de la tumeur mammaire de la souris.

30. Utilisation selon les revendications 26 et 29, dans laquelle la séquence régulatrice contient le fragment PstI à 0,6 Kb du promoteur du virus de la tumeur mammaire de la souris.

31. Utilisation selon les revendications 26 à 30, dans laquelle le gène thérapeutique est sélectionné parmi les gènes anti-tumeur et les gènes de cytokines.

32. Utilisation selon la revendication 31, dans laquelle ledit gène thérapeutique est sélectionné parmi le groupe constitué de gènes qui codent pour les protéines telles que la thymidine-kinase du virus de l'herpès simplex, la cytosine-déaminase, la guanine-phosphoribosyl-transférase (gpt), le cytochrome P 450, de gènes régulateurs de cycle de la cellule qui codent pour des protéines tels que le gène SDI, de gènes suppresseurs de tumeur qui codent pour des protéines tels que le gène p53, de gènes d'antiprolifération qui codent pour des protéines telles que la melittine, la cécropine ou des cytokines telles que l'IL-2.

33. Utilisation selon les revendications 26 à 32, dans laquelle le gène thérapeutique sous contrôle transcriptionnel des séquences régulatrices de la protéine acide du lactosérum ou du virus de la tumeur mammaire de la souris forme une partie d'un vecteur recombiné sélectionné parmi des vecteurs viraux et plasmidiques.

34. Utilisation selon la revendication 33, dans laquelle ledit vecteur viral est sélectionné parmi les vecteurs viraux ARN et ADN et ledit vecteur plasmidique est sélectionné parmi des vecteurs d'expression eucaryotique.

35. Utilisation selon la revendication 34, dans laquelle ledit vecteur viral est un vecteur rétroviral.

36. Utilisation selon la revendication 35, dans laquelle le vecteur rétroviral comprend une région 5'LTR de la structure U3-R-U5 ; au moins une séquence codante codant pour un gène thérapeutique ; et une région 3'LTR comprenant une région U3 complètement ou partiellement délétée, dans laquelle ladite région délétée a été remplacée par un lieur multisite contenant les séquences régulatrices de la protéine acide du lactosérum ou du virus de la tumeur mammaire de la souris suivies par les régions R et U5, ledit gène thérapeutique étant sous contrôle transcriptionnel des séquences régulatrices de la protéine acide du lactosérum ou du virus de la tumeur mammaire de la souris.
